# EUROPÄISCHE PATENTSCHRIFT

(11) **EP 1 280 799 B1**
(45) Veröffentlichungstag und Bekanntmachung des Hinweises auf die Patenterteilung: **21.01.2004**
(21) Anmeldenummer: 01940416.9
(22) Anmeldetag: 08.05.2001
(51) Int. Cl.: C07D 405/12, C07D 401/12, C07D 213/38, A61K 31/44, A61P 35/00

(54) **ORTHO SUBSTITUIERTE ANTHRANILSÄUREAMIDE UND DEREN VERWENDUNG ALS ARZNEIMITTEL**
ORTHO-SUBSTITUTED ANTHRANILIC ACID AMIDES AND THEIR USE AS MEDICAMENTS
AMIDES D'ACIDE ANTHRANILIQUE ORTHO-SUBSTITUES ET LEUR UTILISATION EN TANT QUE MEDICAMENTS

(30) Priorität: 09.05.2000 DE 10023486
(43) Veröffentlichungstag der Anmeldung: 05.02.2003
(73) Patentinhaber: Schering Aktiengesellschaft, 13353 Berlin (DE)
(72) Erfinder: KRÜGER, Martin, 13465 Berlin (DE); HUTH, Andreas, 12437 Berlin (DE); PETROV, Orlin, 14199 Berlin (DE); SEIDELMANN, Dieter, 12159 Berlin (DE); THIERAUCH, Karl-Heinz, 14169 Berlin (DE); HABEREY, Martin, 12169 Berlin (DE); MENRAD, Andreas, 16515 Oranienburg (DE); ERNST, Alexander, 10777 Berlin (DE)
(86) Internationale Anmeldenummer: PCT/EP2001/005214
(87) Internationale Veröffentlichungsnummer: WO 2001/085719

(56) Entgegenhaltungen:
- WO-A-00/27819
- WO-A-00/27820
- AUGUSTIN H G: "Antiangiogenic tumour therapy: will it work?" TRENDS IN PHARMACOLOGICAL SCIENCES, ELSEVIER TRENDS JOURNAL, CAMBRIDGE, GB, Bd. 19, Nr. 6, 1. Juni 1998 (1998-06-01), Seiten 216-222, XP004145666 ISSN: 0165-6147

## Beschreibung

Die Erfindung betrifft ortho substituierte Anthranilsäureamide und deren Verwendung als Arzneimittel zur Behandlung von Erkrankungen, die durch persistente Angiogenese ausgelöst werden.
Persistente Angiogenese kann die Ursache für verschiedene Erkrankungen wie Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen und Artheriosklerose sein oder zu einer Verschlimmerung dieser Erkrankungen führen.
In dem Reviewartikel "Antiangiogenic tumor therapy: will it work?" (Augustin H G, Trends in Pharmacological sciences, Elsevier Trends Journal, Cambridge, Bd 19, Nr. 6, 1. Juni 1998, Seiten 216-222) werden allgemein die Unterschiede in physiologischer Angiogenese in erwachsenem Gewebe und pathologischer Angiogenese in experimentellen Tiertumoren sowie natürlichen menschlichen Tumoren aufgezeigt und experiemtelle Herangehensweisen zur antiangiogenetischen Tumortherapie und deren Umsetzung in die klinische Praxis beschrieben.
Eine direkte oder indirekte Inhibition des VEGF-Rezeptors kann zur Behandlung derartiger Erkrankungen und anderer VEGF-induzierter pathologischer Angiogenese und vaskularer permeabiler Bedingungen, wie Tumor-Vaskularisierung, verwendet werden. Beispielsweise ist bekannt, daß durch lösliche Rezeptoren und Antikörper gegen VEGF das Wachstum von Tumoren gehemmt werden kann.
Die persistente Angiogenese wird durch den Faktor VEGF über seinen Rezeptor induziert. Damit VEGF diese Wirkung entfalten kann ist es nötig, daß VEGF am Rezeptor bindet und eine Tyrosinphosphorylierung hervorgerufen wird.

Es sind bereits Phenyl-Anthranilamid-Derivate bekannt, die als Angiotensin II-Antagonisten (EP 564 356) und als Entzündungshemmer und Anti-Ulcera-Verbindungen (U.S. 3,409,668) zur Anwendung kommen.

Es wurde nun gefunden, daß Verbindungen der allgemeinen Formel I 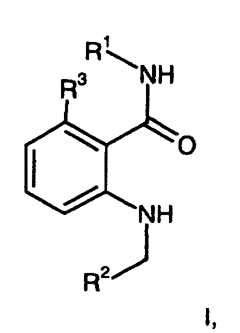 in der 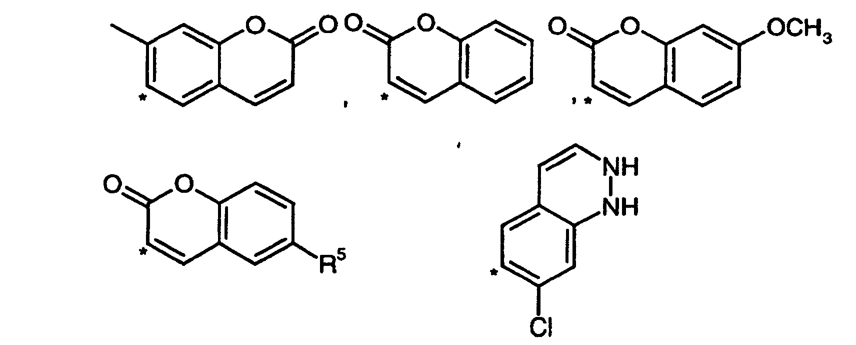 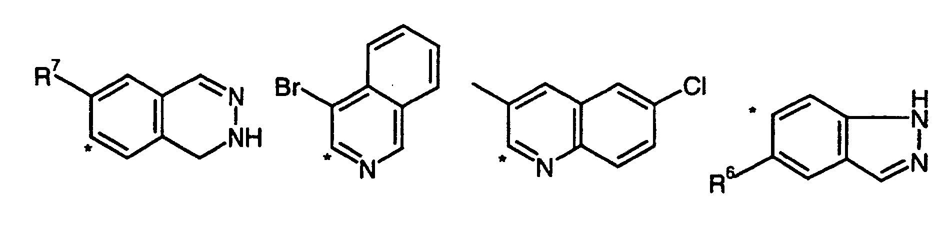 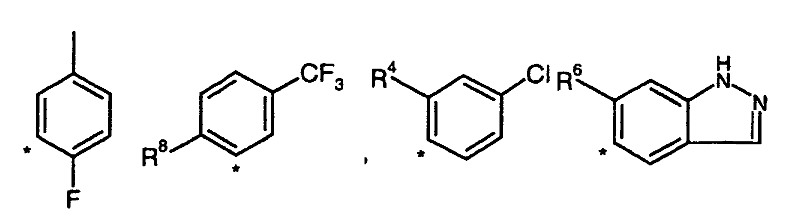 steht,
in der R⁴ Fluor, Chlor, Brom, -CF₃, -N=C, CH₃-,-OCF₃ oder -CH₂OH bedeutet,
in der R⁵ Chlor, Brom oder OCH₃ bedeutet,
in der R⁶ -CH₃ oder Chlor bedeutet,
in der R⁷ CH₃ oder Chlor bedeutet,
in der R⁸ CH₃, Fluor, Chlor oder CF₃ bedeutet,
in der R² für Pyridyl oder die Gruppe 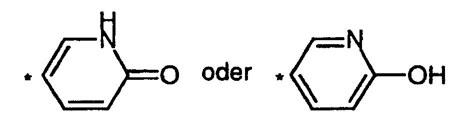 steht und
R³ für Wasserstoff oder Fluor steht, bedeuten
sowie deren Isomeren und Salze,
wobei falls
R¹ für die Gruppe 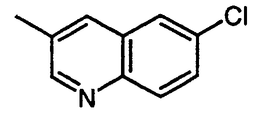 und R³ für Wasserstoff steht, dann darf R² nicht für Pyridyl stehen,
eine Tyrosinphosphorylierung bzw. die persistente Angiogenese stoppen und damit das Wachstum und ein Ausbreiten von Tumoren verhindern.
Es gibt unterschiedliche Tyrosinkinasen (Web Site: S. Hauk, A. M. Quinn, Meth. in Enzymol. 1991, 200, 38-62, speziell in der katalytischen Domäne, Untergruppe PTK group XIV, Web page:
http://www.sdsc.edu/Kinases/pkr/pk catalytic/pk hanks seq align long.html und Mc Tigue et al., Structure 1999, 7, 319-330), die in ähnlicher Weise gehemmt werden können. So wird üblicherweise auch die Tyrosinkinase C, die u. a. in den Stammzellen vorkommt, durch Verbindungen, die VEGF (vaskulärer endotheiiaier Wachtumsfaktor) hemmen, ebenfalls gehemmt. Es ist somit wünschenswert Verbindungen zu haben, die selektiv VEGF hemmen.
Die erfindungsgemäßen Verbindungen zeichnen sich nun gerade dadurch aus, dass sie über derartige selektive Eigenschaften verfügen und somit wertvolle Verbindungen darstellen, die das Wachstum und die Ausbreitung von Tumoren unterbinden.

Die erfindungsgemäßen Verbindungen der allgemeinen Formel I beinhalten auch die möglichen tautomeren Formen und umfassen die E- oder Z-Isomeren oder, falls ein chirales Zentrum vorhanden ist, auch die Racemate und Enantiomeren.

Die Verbindungen der Formel I sowie deren physiologisch verträglichen Salze sind auf Grund ihrer inhibitorischen Aktivität in Bezug auf Phosphorylierung des VEGF-Rezeptors als Arzneimittel verwendbar. Auf Grund ihres Wirkprofils eignen sich die erfindungsgemäßen Verbindungen zur Behandlung von Erkrankungen, die durch eine persistente Angiogenese hervorgerufen werden.

Da die Verbindungen der Formel I als Inhibitoren der Tyrosinkinase KDR und FLT identifiziert werden, eignen sie sich insbesondere zur Behandlung von solchen Krankheiten, die durch die über den VEGF-Rezeptor ausgelöste persistente Angiogenese oder eine Erhöhung der Gefäßpermeabilität hervorgerufen werden.

Gegenstand der vorliegenden Erfindung ist auch die Verwendung der erfindungsgemäßen Verbindungen als Inhibitoren der Tyrosinkinase KDR und FLT.

Gegenstand der vorliegenden Erfindung sind somit auch Arzneimittel zur Behandlung von Tumoren bzw. deren Verwendung.

Die erfindungsgemäßen Verbindungen können entweder alleine oder in Formulierung als Arzneimittel zur Behandlung von Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose und Verletzungen des Nervengewebes zum Einsatz kommen.
Die erfindungsgemäßen Verbindungen können ebenfalls zum Einsatz kommen bei der Hemmung der Reocclusion von Gefäßen nach Ballonkathetherbehandlung, bei der Gefäßprothetik oder nach dem Einsetzen von mechanischen Vorrichtungen zum Offenhalten von Gefäßen wie z. B. Stents.

Bei der Behandlung von Verletzungen des Nervengewebes kann mit den erfindungsgemäßen Verbindungen eine schnelle Narbenbildung an den Verletzungsstellen verhindert werden, d. h. es wird verhindert, daß die Narbenbildung eintritt, bevor die Axone wieder Verbindung miteinander aufnehmen. Damit würde eine Rekonstruktion der Nervenverbindungen erleichtert.
Femer kann mit den erfindungsgemäßen Verbindungen die Ascites-Bildung bei Patienten unterdrückt werden. Ebenso lassen sich VEGF bedingte Ödeme unterdrücken.
Derartige Arzneimittel, deren Formulierungen und Verwendungen sind ebenfalls Gegenstand der vorliegenden Erfindung.

Die Erfindung betrifft ferner die Verwendung der Verbindungen der allgemeinen Formel I, zur Herstellung eines Arzneimittels zur Behandlung von Tumoren, Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose, Verletzungen des Nervengewebes, Hemmung der Reocclusion von Gefäßen nach Ballonkathetherbehandlung, bei der Gefäßprothetik oder nach dem Einsetzen von mechanischen Vorrichtungen zum Offenhalten von Gefäßen wie z. B. Stents.

Zur Verwendung der Verbindungen der Formel I als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.
Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-2000 mg, vorzugsweise 50-1000 mg, wobei die Dosis als einmal zu verabreichende Einzeldosis oder unterteilt in 2 oder mehreren Teildosen gegeben werden kann. Die oben beschrieben Formulierungen und Darreichungsformen sind ebenfalls Gegenstand der vorliegenden Erfindung.
Die Herstellung der erfindungsgemäßen Verbindungen erfolgt nach an sich bekannten Methoden. Beispielsweise gelangt man zu Verbindungen der Formel I dadurch, daß man
a) eine Verbindung der Formel II 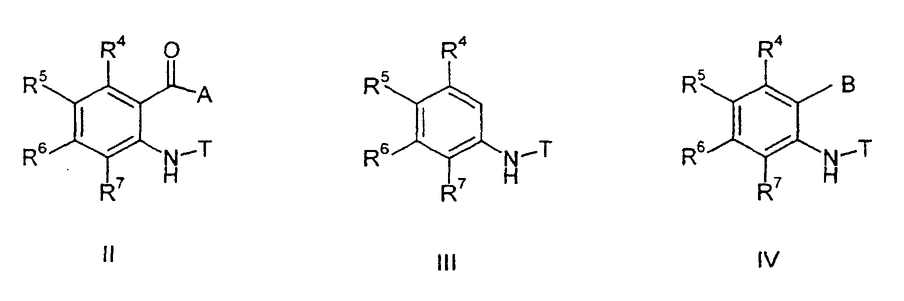 worin R⁴ bis R⁷ die obige Bedeutung haben und T H oder eine Schutzgruppe und A Halogen oder OR¹³ ist, wobei R¹³ ein Wasserstoffatom, C₁₋₄-Alkyl oder C₁₋₄-Acyl bedeutet oder einen Ring mit T schliesst, zunächst N alkyliert und dann COA in ein Amid überführt und dann gegebenenfalls Schutzgruppen abspaltet oder zunächst in daß Amid überführt und anschließend N-alkyliert oder
b) eine Verbindung der Formel III
   worin R⁴ bis R⁷ die obige Bedeutung haben und T H oder eine Schutzgruppe bedeuten orthometalliert und dann durch Abfang mit einem Elektrophil in ein Amid überführt, dann die Schutzgruppe abspaltet und die Aminogruppe alkyliert,
   oder
c) eine Verbindung der Formel IV
worin R⁴ bis R⁷ die obige Bedeutung haben und T H oder eine Schutzgruppe und B Halogen oder O-Triflat, O-Tosylat oder O-Mesylat bedeuten in ein Amid überführt, dann die Schutzgruppe abspaltet und die Aminogruppe alkyliert

Die Reihenfolge der Schritte kann in allen drei Fällen vertauscht werden.

Die Amidbildung erfolgt nach literaturbekannten Methoden.
Zur Amidbildung kann man von einem entsprechenden Ester ausgehen. Der Ester wird nach J. Org. Chem. 1995, 8414 mit Aluminiumtrimethyl und dem entsprechenden Amin in Lösungsmitteln wie Toluol bei Temperaturen von 0°C bis zum Siedepunkt des Lösungsmittels umgesetzt. Diese Methode ist auch bei ungeschützten Anthranilsäureestem anwendbar. Enthält das Molekül zwei Estergruppen, werden beide in das gleiche Amid überführt.

Beim Einsatz von Nitrilen statt des Esters erhält man unter analogen Bedingungen Amidine.

Zur Amidbildung stehen aber auch alle aus der Peptidchemie bekannten Verfahren zur Verfügung. Beispielsweise kann die entsprechende Säure in aprotischen polaren Lösungsmitteln wie zum Beispiel Dimethylformamid über eine aktiviertes Säurederivat, zum Beispiel erhältlich mit Hydroxybenzotriazol und einem Carbodiimid wie zum Beispiel Diisopropylcarbodiimid oder auch mit vorgebildeten Reagenzien wie zum Beispiel HATU (Chem. Comm. 1994, 201, oder BTU, bei Temperaturen zwischen 0°C und dem Siedepunkt des Lösungsmittels vorzugsweise bei 80°C mit dem Amin bei HATU vorzugsweise bei Raumtemperatur umgesetzt werden. Diese Methoden sind auch bei den ungeschützten Anthranilsäuren zu benutzen. Für die Amidbildung kann auch das Verfahren über das gemischte Säureanhydrid, imidazolid oder Azid eingesetzt werden. Ein vorheriger Schutz der Aminogruppe beispielsweise als Amid ist nicht in allen Fällen erforderlich, kann die Reaktion aber günstig beeinflussen. Ein besonderes Ausgangsmaterial sind Isatosäureanhydride, bei denen der Schutz der Aminogruppe und die Aktivierung der Säurefunktion gleichzeitig vorliegen.

Wenn man das Amin vorher in die BOC-geschützte Verbindung überführt, lässt sich die ortho-Stellung durch Umsetzung mit metallorganischen Verbindungen wie beispielsweise n-Butyllithium metallieren und anschliessend mit Isocyanaten oder Isothiocyanaten zu den Anthranilamiden bzw. Anthranilthioamiden abfangen. Ein Brom- oder Jodsubstituent in dieser ortho-Stellung erleichtern durch Halogen-Metall-Austausch die ortho-Metallierung. Als Lösungsmittel eignen sich Ether wie Diethylether oder Tetrahydrofuran oder Kohlenwasserstoffe wie Hexan aber auch Mischungen daraus. Die Zugabe von Komplexbildnern wie Tetramethylethylendiamin (TMEDA) ist vorteilhaft. Die Temperaturen bewegen sich zwischen -78°C und Raumtemperatur. Die Spaltung der BOC-Amide erfolgt durch Behandlung mit Säuren wie Trifluoressigsäure ohne Lösungsmittel oder in Lösungsmitteln wie Methylenchlorid bei Temperaturen von 0°C bis zum Siedepunkt des Lösungsmittels oder mit wässriger Salzsäure vorzugsweise 1N-Salzsäure in

Lösungsmitteln wie Ethanol oder Dioxan bei Temperaturen von Raumtemperatur bis zum Siedepunkt des Lösungsmittels.

Die Amidgruppe kann aber auch durch Carbonylierung eingeführt werden. Dazu geht man von den entsprechenden Verbindungen der Formel IV (o-Jod-, o-Brom- oder o-Triflyloxyanilinen) aus, die mit Kohlenmonoxid bei Normal- oder auch erhöhtem Druck und einem Amin in Gegenwart von Übergangsmetallkatalysatoren wie zum Beispiel Palladium(II)chlorid oder Palladium(II) acetat oder auch Palladiumtetrakistriphenylphosphin in Lösungsmitteln wie Dimethylformamid umgesetzt werden. Die Zugabe eines Liganden wie Triphenylphosphin und die Zugabe einer Base wie Tributylamin kann vorteilhaft sein. (s. beispielsweise J.org.Chem. 1974, 3327; J.org.Chem. 1996, 7482; Synth. Comm. 1997, 367; Tetr.Lett 1998, 2835)

Sollen verschiedene Amidgruppen in das Molekül eingeführt werden, muss beispielsweise die zweite Estergruppe nach der Erzeugung der ersten Amidgruppe in das Molekül eingeführt und dann amidiert werden oder man hat ein Molekül in dem eine Gruppe als Ester, die andere als Säure vorliegt und amidiert die beiden Gruppen nacheinander nach verschiedenen Methoden.

Thioamide sind aus den Anthranilamiden durch Umsetzung mit Diphosphadithianen nach Bull Soc.Chim.Belg. 87, 229,1978 oder durch Umsetzung mit Phosphorpentasulfid in Lösungsmitteln wie Pyridin oder auch ohne Lösungsmittel bei Temperaturen von 0°C bis 200°C zu erhalten..

Die Produkte können als elektronenreiche Aromaten auch elektrophilen aromatischen Substitutionen unterworfen werden. Die Substitution erfolgt dann in der ortho- oder para-Position zu der oder einer der Aminogruppe(n). So kann durch Friedel-Crafts-Acylierung mit Säurechloriden in Gegenwart von Friedel-Crafts Katalysatoren wie zum Beispiel Aluminiumtrichlorid in Lösungsmitteln wie Nitromethan, Schwefelkohlenstoff, Methylenchlorid oder

Nitrobenzol bei Temperaturen zwischen 0°C und dem Siedepunkt des Lösungsmittels vorzugsweise bei Raumtemperatur acyliert werden.

Es kann nach literaturbekannten Verfahren beispielsweise durch Nitriersäure, verschieden konzentrierte Salpetersäure ohne Lösungsmittel oder durch Metalinitrate wie beispielsweise Kupfer(II)nitrat oder Eisen(III)nitrat in polaren Lösungsmitteln wie Ethanol oder Eisessig oder auch in Acetanhydrid eine oder mehrere Nitrogruppen eingeführt werden.

Die Einführung von Halogenen erfolgt nach literaturbekannten Verfahren z.B. durch Umsetzung mit Brom, N-Brom- oder N-Jodsuccinimid oder Urotropinhydrotribromid in polaren Lösungsmitteln wie Tetrahydrofuran, Acetonitril, Methylenchlorid, Eisessig oder Dimethylformamid.

Die Reduktion der Nitrogruppe wird in polaren Lösungsmitteln bei Raumtemperatur oder erhöhter Temperatur durchgeführt. Als Katalysatoren für die Reduktion sind Metalle wie Raney-Nickel oder Edelmetallkatalysatoren wie Palladium oder Platin oder auch Palladiumhydroxid gegebenenfalls auf Trägern geeignet. Statt Wasserstoff können auch zum Beispiel Ammoniumformiat, Cyclohexen oder Hydrazin in bekannter Weise benutzt werden. Reduktionsmittel wie Zinn-II-chlorid oder Titan-(III)-chlorid können ebenso verwendet werden wie komplexe Metallhydride eventuell in Gegenwart von Schwermetallsalzen. Als Reduktionsmittel ist auch Eisen nutzbar. Die Reaktion wird dann in Gegenwart einer Säure wie z.B. Essigsäure oder Ammoniumchlorid gegebenenfalls unter Zusatz eines Lösungsmittels wie zum Beispiel Wasser, Methanol, Eisen/ Ammoniak etc. durchgeführt. Bei verlängerter Reaktionszeit kann bei dieser Variante eine Acylierung der Aminogruppe eintreten.

Wird eine Alkylierung einer Aminogruppe gewünscht, so kann nach üblichen Methoden - beispielsweise mit Alkylhalogeniden - oder nach der Mitsunobu Variante durch Umsetzung mit einem Alkohol in Gegenwart von beispielsweise Triphenylphosphin und Azodicarbonsäureester alkyliert werden. Man kann auch das Amin einer reduktiven Alkylierung mit Aldehyden oder Ketonen unterwerfen, wobei man in Gegenwart eines Reduktionsmittels wie beispielsweise Natriumcyanoborhydrid in einem geeigneten inerten Lösungsmittel wie zum Beispiel Ethanol bei Temperaturen von 0°C bis zum Siedepunkt des Lösungsmittels umsetzt. Wenn man von einer primären Aminogruppe ausgeht, so kann man gegebenenfalls nacheinander mit zwei verschiedenen Carbonylverbindungen umsetzen, wobei man gemischte Derivate erhält [Literatur z.B. Verardo et al. Synthesis (1993), 121; Synthesis (1991), 447; Kawaguchi, Synthesis (1985), 701; Micovic et al. Synthesis (1991), 1043].
Es kann vorteilhaft sein, zunächst die Schiffsche Base durch Umsetzung des Aldehyds mit dem Amin in Lösungsmitteln wie Ethanol oder Methanol, gegebenenfalls unter Zugabe von Hilfsstoffen wie Eisessig zu bilden und dann erst Reduktionsmittel wie z. B. Natriumcyanoborhydrid zuzusetzen.

Die Hydrierung von Alken-oder Alkingruppen im Molekül erfolgt in üblicher Weise beispielsweise durch katalytisch erregten Wasserstoff. Als Katalysatoren können Schwermetalle wie Palladium oder Platin, gegebenenfalls auf einem Träger oder Raney-Nickel benutzt werden. Als Lösungsmittel kommen Alkohole wie z.B. Ethanol in Frage. Es wird bei Temperaturen von 0° C bis zum Siedepunkt des Lösungsmittels und bei Drücken bis zu 20 Bar, vorzugsweise aber bei Raumtemperatur und Normaldruck gearbeitet. Durch die Verwendung von Katalysatoren, wie beispielsweise eines Lindlar-Katalysators lassen sich Dreifachbindungen zu Doppelbindungen partiell hydrieren, wobei vorzugsweise die Z-Form entsteht.

Die Acylierung einer Aminogruppe erfolgt in üblicher Weise beispielsweise mit einem Säurehalogenid oder Säureanhydrid gegebenenfalls in Gegenwart einer Base wie Dimethylaminopyridin in Lösungsmitteln wie Methylenchlorid, Tetrahydrofuran oder Pyridin, nach der Schotten-Baumann-Variante in wäßriger

Lösung bei schwach alkalischem pH-Wert oder durch Umsetzung mit einem Anhydrid in Eisessig.

Die Einführung der Halogene Chlor, Brom, Jod oder der Azidogruppe über eine Aminogruppe kann beispielsweise auch nach Sandmeyer erfolgen, indem man die mit Nitriten intermediär gebildeten Diazoniumsalze mit Kupfer(I)chlorid oder Kupfer(I)bromid in Gegenwart der entsprechenden Säure wie Salzsäure oder Bromwasserstoffsäure oder mit Kaliumjodid umsetzt.

Wenn ein organischer Salpetrigsäureester benutzt wird, kann man die Halogene z.B. durch Zusatz von Methylenjodid oder Tetrabrommethan einführen in einem Lösungsmittel wie zum Beispiel Dimethylformamid. Die Entfemung der Aminogruppe kann entweder durch Umsetzung mit einem organischen Salpetrigsäureester in Tetrahydrofuran oder durch Diazotierung und reduktive Verkochung des Diazoniumsalzes beispielsweise mit phosphoriger Säure gegebenenfalls unter Zugabe von Kupfer (I) oxid bewerkstelligt werden.

Die Einführung von Fluor gelingt beispielsweise durch Balz-Schiemann-Reaktion des Diazoniumtetrafluorborates oder nach J. Fluor. Chem. 76,1996,59-62 durch Diazotierung i.G. von HFxPyridin und anschliessende Verkochung gegebenenfalls i.G. einer Fluoridionenquelle wie z.B. Tetrabutylammoniumfluorid.

Die Einführung der Azidogruppe gelingt nach Diazotierung durch Umsetzung mit Natriumazid bei Raumtemperatur.

Etherspaltungen werden nach literaturüblichen Verfahren durchgeführt. Dabei kann auch bei mehreren im Molekül vorhandenen Gruppen eine selektive Spaltung erreicht werden. Dabei wird der Ether beispielsweise mit Bortribromid in Lösungsmitteln wie Dichlormethan bei Temperaturen zwischen -100 °C bis zum Siedepunkt des Lösungsmittels vorzugsweise bei -78 °C behandelt. Es ist aber auch möglich, den Ether durch Natriumthiomethylat in Lösungsmitteln wie Dimethylformamid zu spalten. Die Temperatur kann zwischen Raumtemperatur und dem Siedepunkt des Lösungsmittels vorzugsweise bei 150°C liegen.

Die N- oder O-Alkylierung von Amiden wie dem Pyrid-2-on bzw 2-Hydroxypyridin gelingt nach literaturbekannten Methoden. So erreicht man mit Basen wie Natriumhydrid oder Kaliumcarbonat in Lösungsmitteln wie Dimethylformamid und Alkylierung mit Alkylhalogeniden wie Methyljodid eine N-Alkylierung. Mit Basen wie Silberkarbonat iin Lösungsmitteln wie Tetrahydrofuran oder Toluol oder Vorzugsweise Mischungen davon mit Alkylhalogeniden wie Methyljodid eine O-Alkylierung. Eine O-Alkylierung erhält man auch beim Umsatz mit Trialkyloxoniumtetrafluoroborat in inerten Lösungsmitteln wie Methylenchlorid. Die Umsetzung mit Diazomethan oder Trimethylsilyldiazomethan in Lösungsmitteln wie Methanol oder Toluol vorzugsweise in Mischungen davon bei Temperaturen bis zum Siedepunkt der Lösungsmittel vorzugsweise aber bei Raumtemperatur erhält man Gemische aus N-und O-Alkylderivaten. Die Methoden ermöglichen eine selektive Alkylierung des Pyridons gegenüber dem Benzoesäureamid.

Die isomerengemische können nach üblichen Methoden wie beispielsweise Kristallisation, Chromatographie oder Salzbildung in die Enantiomeren bzw. E/Z-Isomeren aufgetrennt werden.

Die Herstellung der Salze erfolgt in üblicher Weise, indem man eine Lösung der Verbindung der Formel mit der äquivalenten Menge oder einem Überschuß einer Base oder Säure, die gegebenenfalls in Lösung ist, versetzt und den Niederschlag abtrennt oder in üblicher Weise die Lösung aufarbeitet.

Die nachfolgenden Beispiele erläutern die Herstellung der erfindungsgemäßen selektiven Verbindungen.

### Beispiel 1

### N-[2-Oxo-2H-1-benzopyran-3-yl-]-2-[(4-pyridyl)methyl]amino-benzoesäureamid

1. 484 mg 3-Amino-2-oxo-2H-1-benzopyran werden in 20 ml Methylenchlorid vorgelegt. Unter Eiskühlung werden 0,42 ml Triethylamin und 0,40 ml 2-Nitrobenzoylchlorid zugetropft. Es wird 4 Stunden bei Raumtemperatur gerührt, das Lösungsmittel abdestilliert, der Rückstand mit Natriumhydrogencarbonat-Lösung aufgenommen und das Produkt abgesaugt. Man erhält 0,88 g N-[2-Oxo-2H-1-benzopyran-3-yl-]-2-nitrobenzoesäureamid.3-Amino-2-oxo-2H-1-benzopyran wurde nach Bonsignore und Loy, J. Heterocyclic Chem., 35, 117 (1998) aus 2-Oxo-2H-1-benzopyran-3-carbonsäure (J. Org. Chem., 64, 1033-1035 (1999)) hergestellt.
2. 880 mg N-[2-Oxo-2H-1-benzopyran-3-yl-]-2-nitro-benzoesäureamid werden in 30 ml Ethanol unter Stickstoff vorgelegt und mit 11 ml Cyclohexen und 176 mg Palladiumhydroxid auf Kohle versetzt und 2 Stunden bei 110 °C gerührt. Der Katalysator wird abfiltriert und das Filtrat bis fast zur Trockene eingeengt. Das Produkt wird abgesaugt. Man erhält 593 mg N-[2-Oxo-2H-1-benzopyran-3-yl-]-2-amino-benzoesäureamid.
3. 645 mg N-[2-Oxo-2H-1-benzopyran-3-yl-]-2-amino-benzoesäureamid in 50 ml Methanol und 170 ml Eisessig werden bei Raumtemperatur vorgelegt und mit 0,38 ml 4-Pyridincarbaldehyd versetzt. Die Mischung wird 15 Stunden gerührt und anschließend mit 206 mg Natriumcyanoborhydrid versetzt. Es wird 24 Stunden gerührt und die Kristalle abgesaugt. Die Kristalle werden mit 70 ml Methanol, 40 ml Eisessig und 95 □l 4-Pyridincarbaldehyd versetzt und 48 Stunden gerührt. Es werden 57 mg Natriumcyanoborhydrid hinzugefügt und 15 Stunden gerührt. Das Produkt wird abgesaugt, mit Methanol gewaschen und getrocknet. Man erhält 521 mg N-[2-Oxo-2H-1-benzopyran-3-yl-]-2-[(4-pyridyl)methyl]amino-benzoesäureamid vom Schmelzpunkt 195-197 °C.

### Beispiel 2

### N-(6-Chlorindazol-5-yl)-2-[(4-pyridyl)methyl]amino-benzoesäureamid

194 mg (0,85mMol) 2-(4-Pyridylmethyl)aminobenzoesäure werden in 8 ml Dimethylformamid mit 283 mg (1,69mMol) 5-Amino-6-chlorindazol versetzt. Zu dieser Lösung werden unter Argon und Feuchtigkeitsausschluß 215 mg (2,13mMol) N-Methylmorpholin und 386 mg (1,02mMol) O-(7-Azabenzotriazol-1-yl)-1,1,3,3-tetramethyluroniumhexafluorophosphat hinzugefügt. Diese Mischung wird 4 Stunden bei Raumtemperatur gerührt. Man verdünnt dann mit ca 40 ml Wasser und extrahiert dreimal mit je 30 ml Ethylacetat. Die vereinigte organische Phase wird mit Wasser gewaschen, getrocknet, filtriert und eingeengt. Der Rückstand wird über Kieselgel mit Methylenchlorid:Ethanol=1 0:1 als Elutionsmittel chromatographiert. Man erhält 97 mg (30,2% d.Th.) an N-(6-Chlorindazol-5-yl)-2-[(4-pyridyl)methyl]amino-benzoesäureamid vom Schmelzpunkt 222,8 °C.

In analoger Verfahrensweise werden hergestellt:

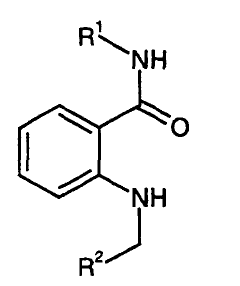

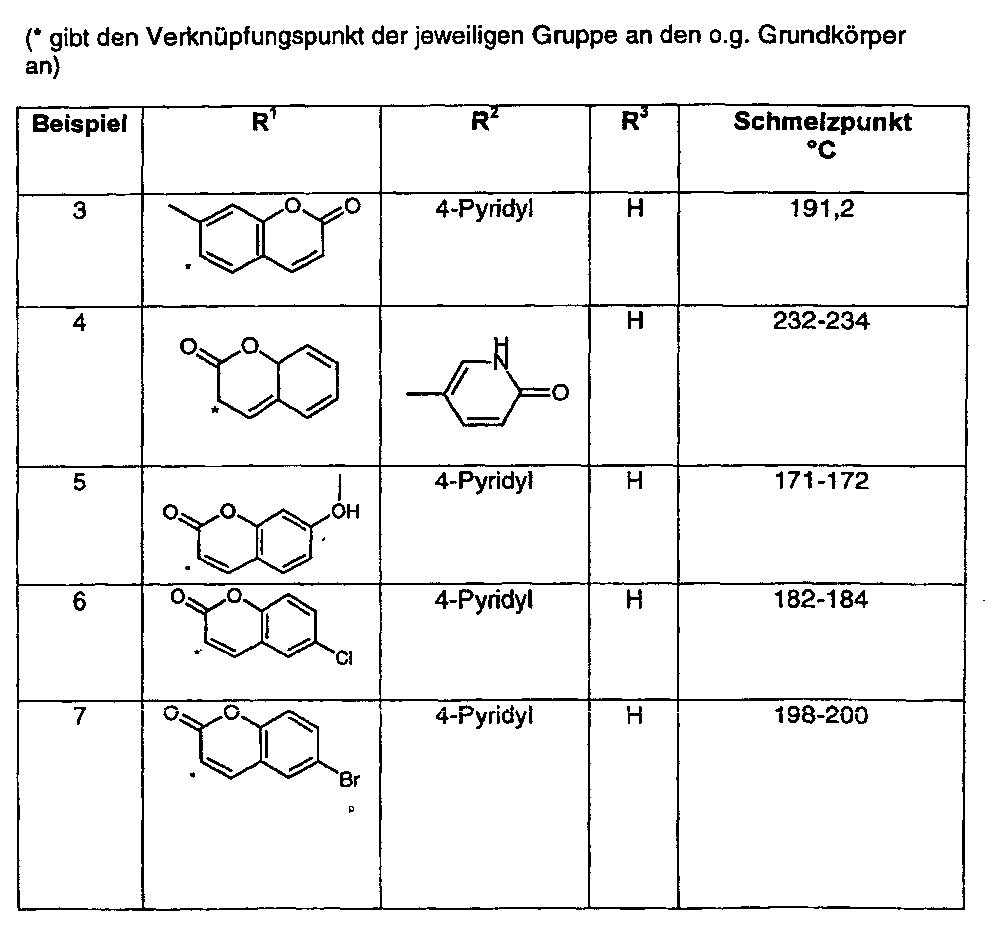

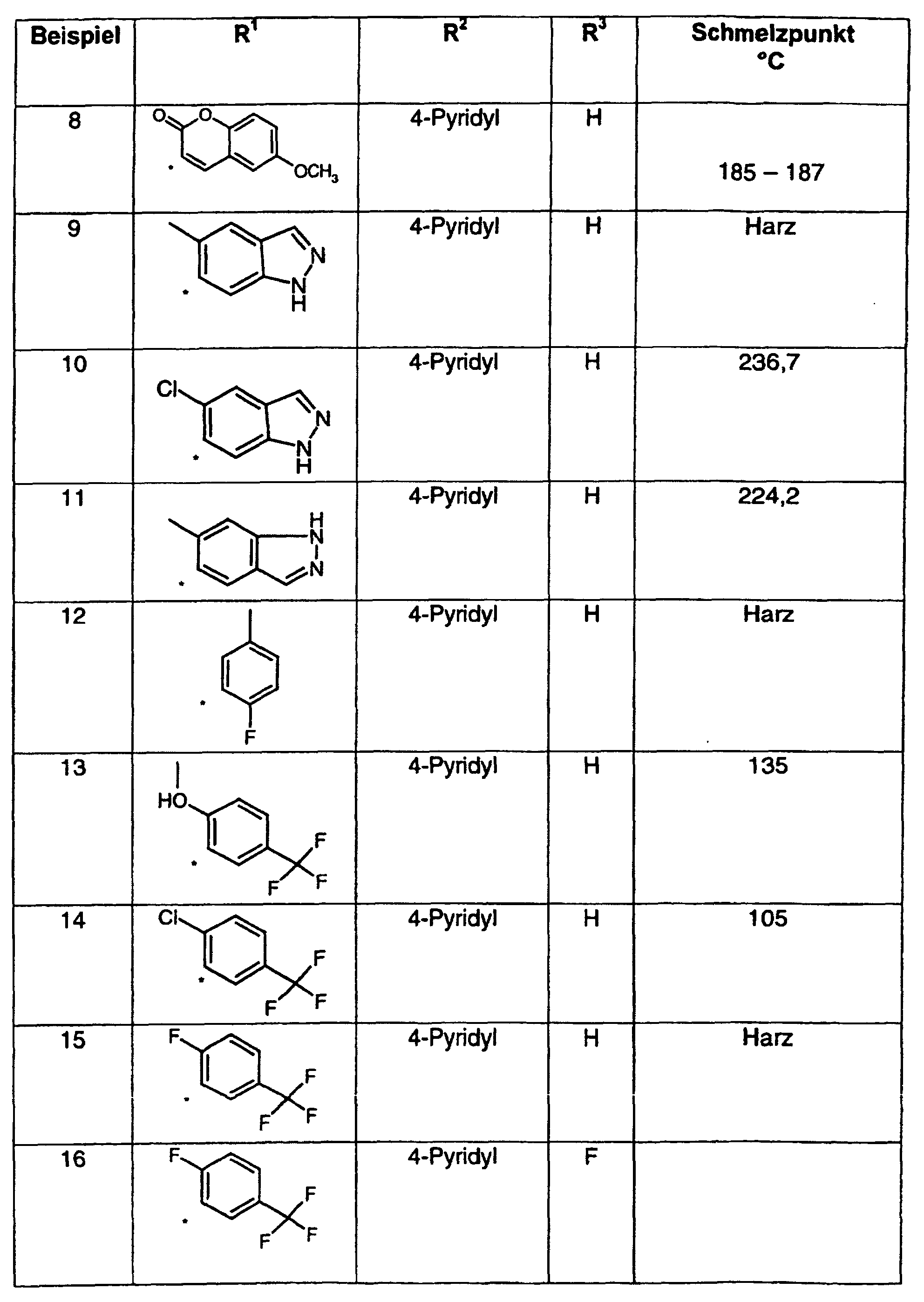

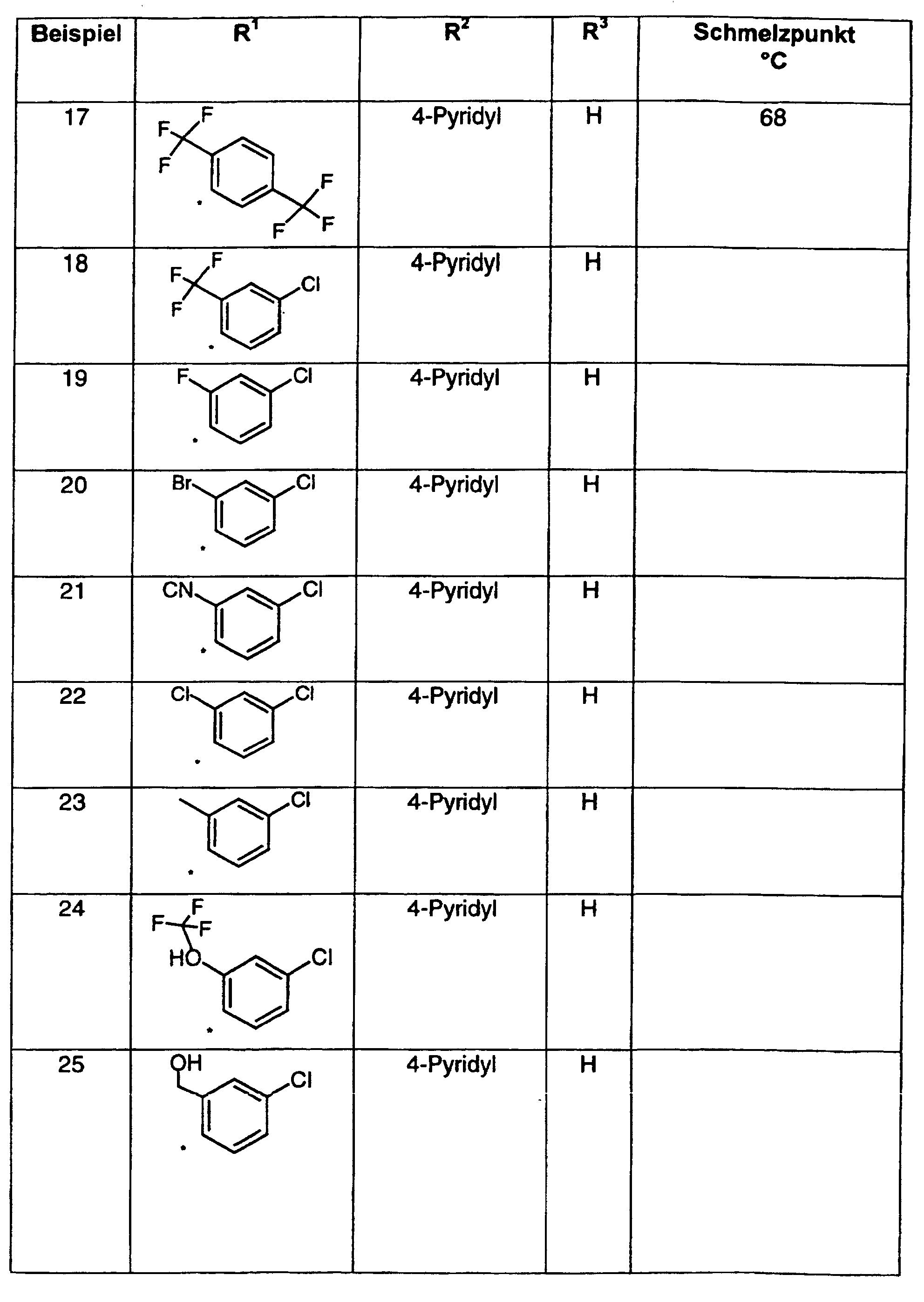

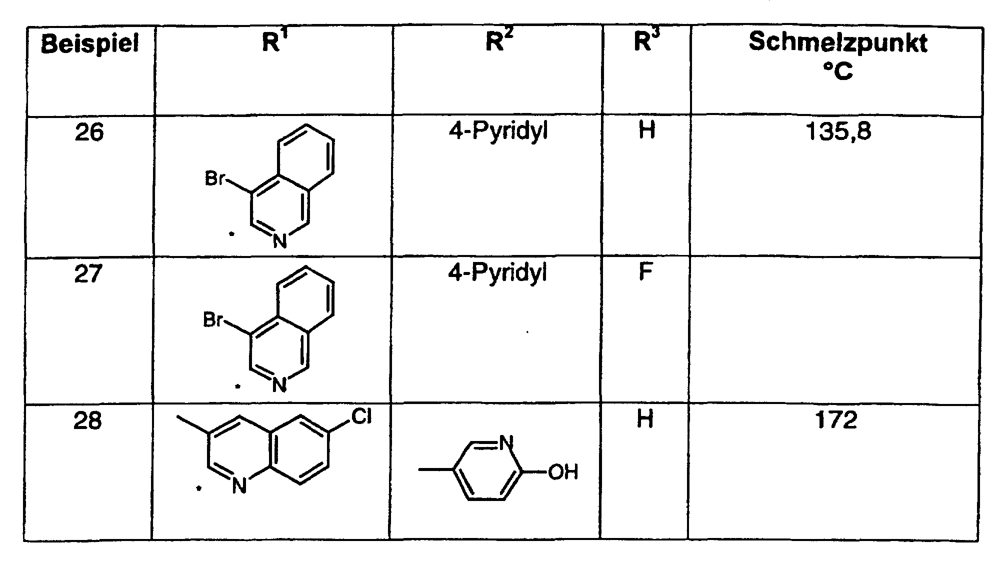

Die nachfolgenden Anwendungsbeispiele erläutem die biologische Wirkung und Verwendung der erfindungsgemäßen selektiven Verbindungen.

### Für die Versuche benötigte Lösungen

| Stammlösungen | |
|---|---|
| Stammlösung A | 3mM ATP in Wasser pH 7,0 (-70°C) |
| Stammlösung B | g-33P-ATP 1mCi/ 100µl |
| Stammlösung C | poly-(Glu4Tyr) 10mg/ ml in Wasser |

| Lösung für Verdünnungen | |
|---|---|
| Substratlösemittel | 10mM DTT, 10 mM Manganchlorid, 100 mM Magnesiumchlorid |
| Enzymlösung | 120 mM Tris/ HCl, pH 7,5, 10 µM Natriumvanadiumoxid |

### Anwendungsbeispiel 1

### Hemmung der KDR-Kinaseaktivität in Gegenwart der erfindungsgemäßen Verbindungen

In einer spitz zulaufenden Mikrotiterplatte (ohne Proteinbindung) werden 10 µl Substratmix (10µl Vol ATP Stammlösung A + 25µCi g-33P-ATP (ca. 2,5 µl der Stammlösung B) + 30µl poly-(Glu4Tyr) Stammlösung C + 1,21ml Substratlösemittel), 10 µl Hemmstofflösung (Substanzen entsprechend den Verdünnungen, als Kontrolle 3% DMSO in Substratlösemittel) und 10 µl Enzymlösung (11,25µg Enzymstammlösung (KDR oder FLT-1 Kinase) werden bei 4°C in 1,25ml Enzymlösung verdünnt) gegeben. Es wird gründlich durchgemischt und bei 10 Minuten Raumtemperatur inkubiert. Anschließend gibt man 10µl Stop-Lösung (250mM EDTA, pH 7,0) zu, mischt und überträgt 10 µl der Lösung auf einen P 81 Phosphozellulosefilter. Anschließend wird mehrfach in 0,1 M Phosphorsäure gewaschen. Das Filterpapier wird getrocknet, mit Meltilex beschichtet und im Microbetazähler gemessen. Die IC50-Werte bestimmen sich aus der Inhibitorkonzentration, die notwendig ist, um den Phosphateinbau auf 50% des ungehemmten Einbaus nach Abzug des Leerwertes (EDTA gestoppte Reaktion) zu hemmen.

Die Ergebnisse der Kinase-Inhibition IC50 in µM sind in der nachfolgenden Tabelle dargestellt.

### Anwendungsbeispiel 2

| C-Kit isolierter Kinase-Inhibitions-Test | |
|---|---|
| Stamm-Lösungen | |
| 10fach Lösungsmittel | 400 mM TrisHCl pH 7,5; 10 mM DTT, 10 mM Manganchlorid, 100 mM Magnesiumchlorid, 2,5 ‰ Polyethyleneglykol 20 000. |
| Inhibitoren | 2 mM in Dimethylsulfoxid |
| | |
| Substrat-Stamm-Lösung | 3 mg/ml Poly (Glu4Tyr)n Sigma P275 in Wasser, gefrorene Anteile |
| | |
| ATP-Stamm-Lösung | 37,5 µM ATP in Wasser, eingestellt auf pH 7.5 und gefrorene Anteile. |
| | |
| 100fach Vanadat-Lösung | 1 mM Natriumvanadat in Wasser |
| | |
| Stop-Lösung | 250 mM Ethylentetraaminoessigsäure (EDTA), pH 7.0 |
| | |
| Filter-Wasch-Lösung | 0,5% Phosphorsäure |

### Assay-Lösungen

### Substrat-Lösungen für 125 Assays:

10 µl ATP (37,5 µM), 25 µCi γ33P-ATP (∼+2,5 µl of Amersham redivue solution) and 10 µl poly-(glu,tyr) werden mit 1,23 ml Lösungsmittel (1mal) gemischt.

### Inhibitor-Lösung:

Die Verbindungen werden im Lösungsmittel (1fach) auf die gewünschte Konzentration gebracht.

### Enzym-Lösung:

Die entsprechende enzymatische Präparation wird mit dem Lösungsmittel (1fach) auf die notwendige Konzentration gebracht, um 1,24 ml zu erhalten. Anschließend werden 12,5 µl Natriumvanadat-Lösung zugegeben.

### Assay

Die Bestandteile werden auf eine Mikrotiterplatte mit runden oder punktierten Böden in der folgenden Reihenfolge gegeben:
10 µl des Inhibitors in dreifacher Endkonzentration,
10 µl Substratmischung
mischen und starten der Reaktion durch Hinzufügen von:
10 µl Enzym-Präparation

Der Ansatz wird 10 Minuten inkubiert und anschließend durch Hinzufügen von 10 µl Stop-Lösung abgebrochen. 10 µl des so behandelten Ansatzes werden auf Phosphocellulose-Filter gegeben, in Phosphorsäure gewaschen wash, anschließend getrocknet und dann in einem meltilex Szintillator geschmolzen und gemessen.

| **Beispiel** | **KDR** **IC50 (µmol/l)** | **C-Kit** **IC50 (µmol/l)** |
|---|---|---|
| 1 | 0,003 | 6 |
| 2 | 0,2 | >10 |
| 3 | 0,03 | 8 |
| 4 | 0,03 | >10 |
| 5 | 0,01 | >10 |
| 6 | 0,2 | 10 |
| 7 | 0,2 | >10 |
| 9 | 0,04 | 10 |
| 10 | 0,01 | >10 |
| 11 | 1 | >10 |
| 13 | 0,05 | 5 |
| 14 | 0,001 | 5 |
| 15 | 0,002 | 2 |
| 17 | KH | >10 |
| 26 | 0,001 | 2 |
| 28 | 0,05 | 0,5 |
| 29 | 0,2 | >10 |
| 30 | 0,02 | 10 |

## Patentansprüche

1. Verbindungen der allgemeinen Formel I 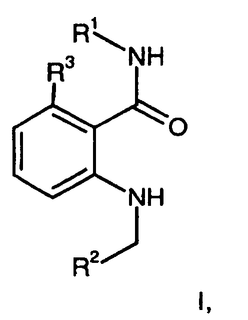 in der
R¹ für die Gruppe 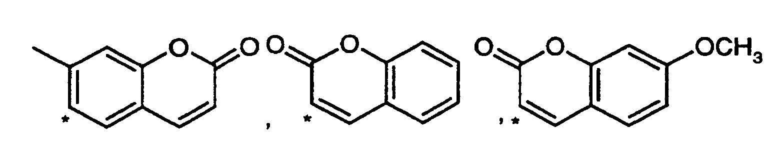 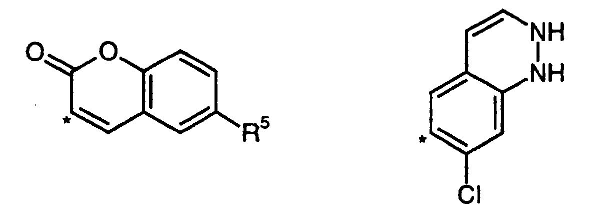 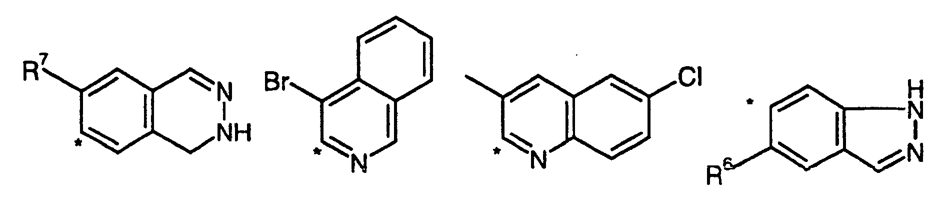 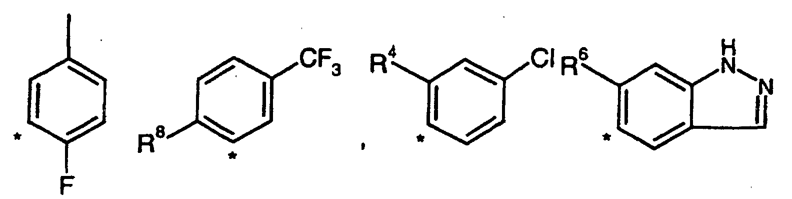 steht,
wobei * den Verknüpfungspunkt angibt
in der R⁴ Fluor, Chlor, Brom, -CF₃, -N=C, CH₃-,-OCF₃ oder -CH₂OH bedeutet,
in der R⁵ Chlor, Brom oder OCH₃ bedeutet,
in der R⁶ -CH₃ oder Chlor bedeutet,
in der R⁷ CH₃ oder Chlor bedeutet,
in der R⁸ CH₃, Fluor, Chlor oder CF₃ bedeutet,
in der R² für Pyridyl oder die Gruppe 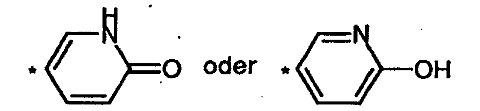 steht und
R³ für Wasserstoff oder Fluor steht, bedeuten,
sowie deren Tautomere, E- oder Z-Isomere, Racemate, Enantiomere und Salze
wobei falls R¹ für die Gruppe 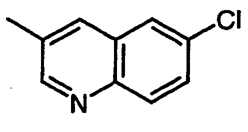 und R³ für Wasserstoff steht, dann darf R² nicht für Pyridyl stehen.

2. Verwendung der Verbindungen der allgemeinen Formel I, gemäß Anspruch 1, zur Herstellung eines Arzneimittels zur Behandlung von Tumoren, Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsab-stoßungen und Glomerulopathie, fibrotische Erkrankungen, wie Leberzirrhose, mesangialzellproliferative Erkrankungen, Artheriosklerose, Verletzungen des Nervengewebes und zur Hemmung der Reocclusion von Gefäßen nach Ballonkathetherbehandlung, bei der Gefäßprothetik oder nach dem Einsetzen von mechanischen Vorrichtungen zum Offenhalten von Gefäßen wie z. B. Stents.

3. Arzneimittel, enthaltend mindestens eine Verbindung gemäß Anspruch 1.

4. Arzneimittel gemäß Anspruch 3, zur Behandlung von Tumoren, Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose, Verletzungen des Nervengewebes und zur Hemmung der Reocclusion von Gefäßen nach Ballonkathetherbehandlung, bei der Gefäßprothetik oder nach dem Einsetzen von mechanischen Vorrichtungen zum Offenhalten von Gefäßen wie z. B. Stents.

5. Verbindungen gemäß Anspruch 1 und Arzneimittel gemäß den Ansprüchen 3 und 4 mit geeigneten Formulierungs und Trägerstoffen.

6. Verwendung der Verbindungen der Formel I gemäß Anspruch 1, als Inhibitoren der Tyrosinkinase KDR und FLT.

7. Verwendung der Verbindungen der allgemeinen Formel I gemäß den Ansprüchen 1 bis 5 in die Form eines pharmazeutischen Präparats für die enteral, parenterale und orale Applikation.

## Claims

1. Compounds of the general formula I 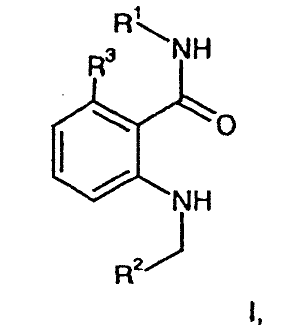 in which
R¹ stands for the group 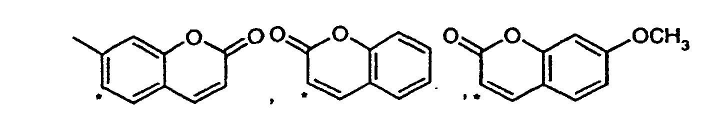 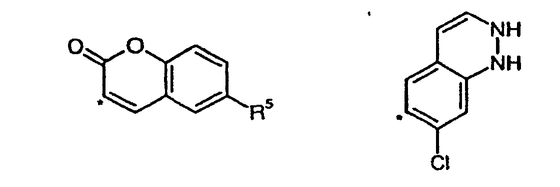 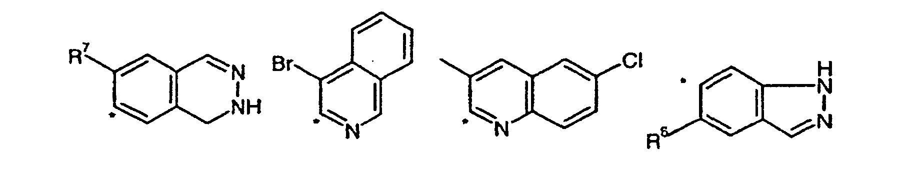 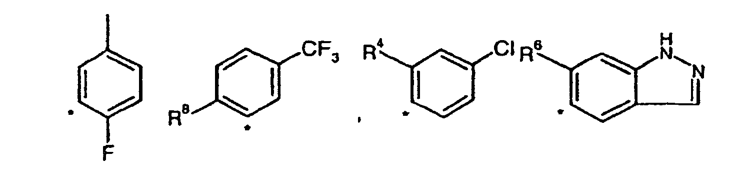 where * indicates the linkage point,
in which R⁴ denotes fluorine, chlorine, bromine, -CF₃, -N=C, CH₃-, -OCF₃ or -CH₂OH,
in which R⁵ denotes chlorine, bromine or OCH₃,
in which R⁶ denotes -CH₃ or chlorine,
in which R⁷ denotes CH₃ or chlorine,
in which R⁸ denotes CH₃, fluorine, chlorine or CH₃,
in which R² stands for pyridyl or the group 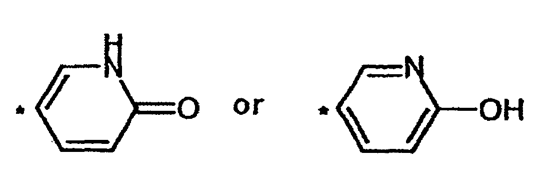 and
R³ stands for hydrogen of fluorine,
and also their tautomers, E isomers or Z isomers, racemates, enantiomers and salts,
where if
R¹ stands for the group 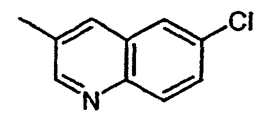 and R³ stands for hydrogen then R² cannot stand for pyridyl.

2. Use of the compounds of the general formula I, according to Claim 1, for preparing a medicament for treating tumours, psoriasis, arthritis, such as rheumatoid arthritis, haemangioma, angiofibroma, eye disorders, such as diabetic retinopathy, neovascular glaucoma, kidney disorders, such as glomerulonephritis, diabetic nephropathy, malignant nephrosclerosis, thrombic microangiopathic syndromes, transplant rejections and glomerulopathy, fibrotic disorders, such as cirrhosis of the liver, mesangial cell proliferation disorders, arteriosclerosis, injuries to the nerve tissue, and for inhibition of the reocclusion of vessels following balloon catheter treatment, in vascular prosthesis or following the insertion of mechanical devices to hold vessels open, such as stents, for example.

3. Medicaments comprising at least one compound according to Claim 1.

4. Medicaments according to Claim 3 for treating tumours, psoriasis, arthritis, such as rheumatoid arthritis, haemangioma, angiofibroma, eye disorders, such as diabetic retinopathy, neovascular glaucoma, kidney disorders, such as glomerulonephritis, diabetic nephropathy, malignant nephrosclerosis, thrombic microangiopathic syndromes, transplant rejections and glomerulopathy, fibrotic disorders, such as cirrhosis of the liver, mesangial cell proliferation disorders, arteriosclerosis, injuries to the nerve tissue, and for inhibition of the reocclusion of vessels following balloon catheter treatment, in vascular prosthesis or following the insertion of mechanical devices to hold vessels open, such as stents, for example.

5. Compounds according to Claim 1 and medicaments according to Claims 3 and 4 with suitable formulating substances and excipients.

6. Use of the compounds of the formula I according to Claim 1 as inhibitors of tyrosine kinase KDR and FLT.

7. Use of the compounds of the general formula I according to Claims 1 to 5 in the form of a pharmaceutical product for enteral, parenteral and oral administration.

## Revendications

1. Composés de la formule générale I 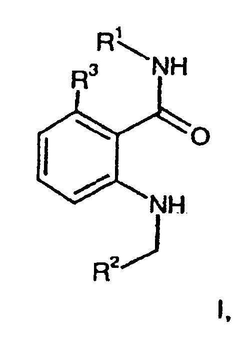 dans laquelle
R¹ représente le groupement 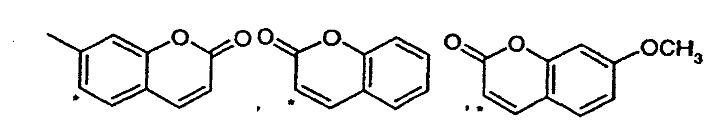 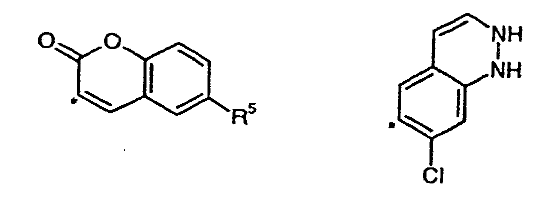 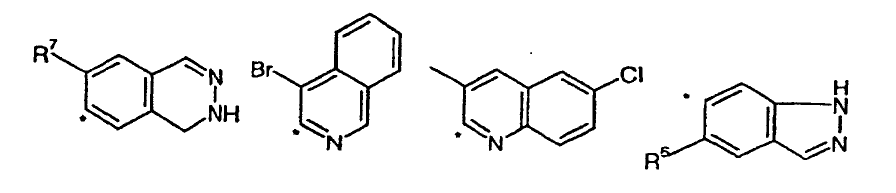 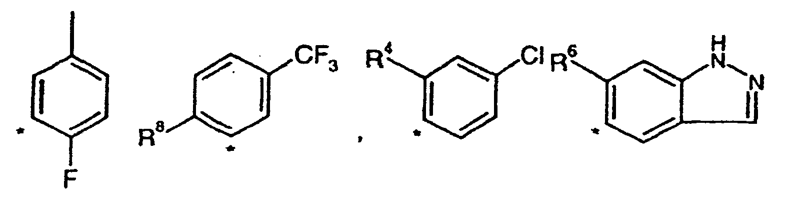 * indiquant le point de connexion,
dans laquelle R⁴ signifie le fluor, le chlore, le brome les radicaux -CF₃, -N=C, CH₃-, -OCF₃ ou -CH₂OH,
dans laquelle R⁵ signifie le chlore, le brome ou le radical OCH₃,
dans laquelle R⁶ signifie le radical -CH₃ ou le chlore,
dans laquelle R⁷ signifie le radical CH₃ ou le chlore,
dans laquelle R⁸ signifie le radical CH₃, le fluor, le chlore ou le radical CF₃,
dans laquelle R² représente le radical pyridyle ou le groupement 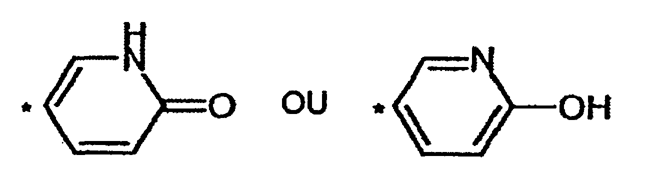
et R³ représente l'hydrogène ou le fluor,
ainsi que leurs tautomères, leurs isomères E ou Z, leurs mélanges racémiques, leurs énantiomères, et leurs sels,
étant donné, si R¹ représente le groupe 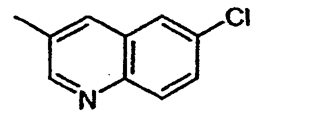 et R³ représente l'hydrogène, R² ne peut, alors, pas représenter le radical pyridyle.

2. Utilisation des composés de la formule générale 1, selon la revendication 1, en vue de la fabrication d'un médicament en vue du traitement de tumeurs, du psoriasis, de l'arthrite, comme l'arthrite rhumatoïde, l'hémangiome, l'angiofibrome, les maladies oculaires, comme la rétinopathie du diabète, le glaucome néovasculaire, les maladies du rein, comme la néphrite glomérulosique, la néphropathie du diabète, les néphroscléroses malignes, les syndromes microangiopatiques thrombotiques, les rejets de transplantation et la glomérulopathie, les maladies fibreuses, comme la cirrhose du foie, les maladies prolifératives des cellules mésangiales, l'artérioscléreuse, les lésions du système nerveux et en vue de l'inhibition de la réocclusion de vaisseaux par un traitement au cathéter à ballon, lors de la prothétique des vaisseaux ou après l'utilisation de dispositifs mécaniques en vue du maintien en position ouvertes de vaisseaux, comme, par exemple, des stents.

3. Médicament, contenant au moins un composé selon la revendication 1.

4. Médicament selon la revendication 3, en vue du traitement de tumeurs, du psoriasis, de l'arthrite, comme l'arthrite rhumatoïde, l'hémangiome, l'angiofibrome, les maladies oculaires, comme la rétinopathie du diabète, le glaucome néovasculaire, les maladies du rein, comme la néphrite glomérulosique, la néphropathie du diabète, les néphroscléroses malignes, les syndromes microangiopatiques thrombotiques, les rejets de transplantation et la glomérulopathie, les maladies fibreuses, comme la cirrhose du foie, les maladies prolifératives des cellules mésangiales, l'artériosclérose, les lésions du système nerveux et en vue de l'inhibition de la ré occlusion de vaisseaux par un traitement au cathéter à ballon, lors de la prothétique des vaisseaux ou après l'utilisation de dispositifs mécaniques en vue du maintien en position ouvertes de vaisseaux, comme, par exemple, des stents.

5. Composés selon la revendication 1 et médicament selon les revendications 3 et 4 avec des formulations et des excipients appropriés.

6. Utilisation des composés de la formule 1 selon la revendication 1, en tant qu'agents inhibiteurs de la tyrosinkinase KDR et FLT.

7. Utilisation des composés de la formule générale I selon les revendications 1 à 5 sous la forme d'une préparation pharmaceutique en vue de l'application entérale, parentérale et orale.
